# EUROPÄISCHE PATENTSCHRIFT

(11) **EP 1 139 991 B1**
(45) Veröffentlichungstag und Bekanntmachung des Hinweises auf die Patenterteilung: **28.08.2002**
(21) Anmeldenummer: 99965478.3
(22) Anmeldetag: 16.12.1999
(51) Int. Cl.: A61K 7/13, D06P 1/18

(54) **MITTEL ZUM FÄRBEN VON KERATINHALTIGEN FASERN**
AGENT FOR DYING KERATIN FIBERS
AGENT POUR LA COLORATION DE FIBRES KERATINIQUES

(30) Priorität: 23.12.1998 DE 19859720
(43) Veröffentlichungstag der Anmeldung: 10.10.2001
(73) Patentinhaber: Henkel Kommanditgesellschaft auf Aktien, 40589 Düsseldorf (DE)
(72) Erfinder: MÖLLER, Hinrich, D-40789 Monheim (DE); MEINIGKE, Bernd, D-51381 Leverkusen (DE)
(86) Internationale Anmeldenummer: EP9909999
(87) Internationale Veröffentlichungsnummer: WO00038637

(56) Entgegenhaltungen:
- WO-A-91/09588
- DE-B- 1 212 683
- FR-A- 1 164 505
- GB-A- 1 308 798
- GB-A- 2 041 010

## Beschreibung

Die Erfindung betrifft ein Mittel zum Färben von keratinhaltigen Fasern, insbesondere menschlichen Haaren, das bestimmte Naphthyl-azo-Verbindungen enthält, die Verwendung dieser Verbindungen als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e. V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i. a. unter dem Einfluß von Oxidationsmitteln wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die Verwendung der unten näher beschriebenen Kombination aus Z-Hydroxy-4-sulfo-1-naphthyl-azo-Verbindungen mit speziellen Metallsalzen der 2. oder 3. Hauptgruppe des Periodensystems und/oder Salzen der Übergangsmetalle zum Färben von keratinhaltigen Fasern wird in der Druckschrift GB 2 041 010 A in einem Färbeverfahren beschrieben, in welchem die Faser einer Wasserdampfbehandlung bei 98-115° C unterzogen wird.

In der Druckschrift GB 1 308 798 A wird ein Verfahren zum Färben von menschlichen Haaren offenbart, in dem eine wässrige Zusammensetzung, enthaltend 0,5-40 Gew.% eines direktziehenden Farbstoffs, 0,5-40 Gew.% eines Konservierungsmittels und 0,2-5 Gew.% eines Verdickers, Verwendung finden.

In der Druckschrift WO 91/09588 A1 werden schaumförmige Haartönungsmittel offenbart, die als Farbstoffe zwingend 4-N-Ethyl-1,4-bis(2-(hydroxyethylamino)-2-nitrobenzol, 4-(2-Ureidoethylamino)-nitrobenzol und 4-(2-Hydroxyethylamino)-3-nitrotoluol sowie gegebenenfalls weitere direkt ziehende Farbstoffe enthalten.

Aufgabe der vorliegenden Erfindung ist es, Färbemittel für Keratinfasern, insbesondere für menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten 2-Hydroxy-4-sulfo-1-naphthyl-azo-Verbindungen allein sowie in Kombination mit bestimmten Metallsalzen sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agenzien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend Naphthyl-azo-Verbindungen mit der Formel I, 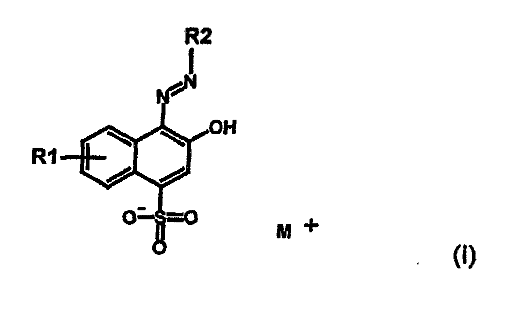
- in der R¹: für Wasserstoff, Halogen, Hydroxy, eine C₁-C₄-Alkylgruppe, eine C₁-C₄-Alkoxygruppe, Sulfogruppe oder Carboxygruppe steht,
- R²: für eine 1- oder 2-Naphthylgruppe, die mindestens eine Hydroxygruppe trägt und als weitere Substituenten Hydroxy-, Sulfo-, Carboxy-, Nitrogruppen oder Halogenatome aufweisen kann, oder für einen 1-Aryl-5-hydroxy-4-pyrazolylrest, der in 3-Stellung durch eine C₁-C₄-Gruppe substituiert sein kann, steht,
- M: für Wasserstoff, ein Alkali- oder 1/2 Erdalkalimetall, 1/3 Al(III), 1/2 Zn(II), 1/4 Ti(IV), 1/4 Zr(IV), 1/3 Cr(III), 1/2 Co(II), 1/2 Fe(II), 1/3 Mo(III) bzw. 1/6 Mo(VI), 1/3 W(III) bzw. 1/6 W(VI) oder Ammonium steht.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z.B. Polyamid-, Polyacrylnitril-, Polyurethan- und Polyesterfasern, verwendet werden.

Vorzugsweise sind die Verbindungen mit der Formel I ausgewählt aus der Gruppe folgender Azoverbindungen: Eriochrom®blauschwarz R, Hydroxynaphtholblau, Acid Blue 158, Eriochrom®rot B.

Diese Substanzen sind literaturbekannt oder im Handel erhältlich.

Die voranstehend genannten Azoverbindungen mit der Formel I sind in den erfindungsgemäßen Mitteln üblicherweise in einer Menge von 0,03 bis 65 mmol, insbesondere von 1 bis 40 mmol, bezogen auf 100 g des gesamten Färbemittels, enthalten.

Sie können als direktziehende Färbemittel oder in Gegenwart von weiteren Komponenten eingesetzt werden.

Färbemittel, die die Azoverbindung mit der Formel I allein enthalten, werden bevorzugt für Färbungen im Bereich von rot, rotviolett, blauviolett und rotbraun eingesetzt.

Eine Ausweitung der Farbnuancen und Verbesserung der Intensität kann erreicht werden, wenn die Azoverbindungen mit der Formel I in Kombination mit Metallsalzen der 2. und 3. Hauptgruppe des Periodensystems und/oder Salzen der Übergangsmetalle eingesetzt werden. Die Verbindungen mit der Formel I und die Metallsalze werden in dieser Ausführungsform vorzugsweise in einem Molverhältnis von 0,5:1 1 bis 2:1 1 eingesetzt.

Die vorzugsweise eingesetzten Metallsalze sind vorzugsweise ausgewählt aus den Chloriden, Sulfaten, Acetaten, Lactaten und Nitraten von Ca, Mg, Al(III), Zn(II), Ti(IV), Zr(IV), Cr(III), Co(II), Fe(II), Mo(III, VI), W(III, VI).

Auf die Anwesenheit von Oxidationsmitteln, z. B. H₂O₂, kann dabei verzichtet werden. Es kann jedoch u.U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche Oxidationsfarbstoffvorprodukte, insbesondere ausgewählt aus den oben genannten Verbindungen, und/oder direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die eingesetzten Komponenten jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkytglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller Dow Corning; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM®-2059 (Hersteller: General Electric), SLM®-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide,
- hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammoniumoder Alkalimetallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von NH₄⁺, Kalium, Natrium oder Lithium, wobei Natriumacetat, Lithiumbromid, Ammoniumcarbonat, -chlorid und -acetat bevorzugt sind. Diese Salze sind vorzugsweise in einer Menge von 0,03 bis 65, insbesondere von 1 bis 40, mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Ein weiterer Gegenstand der vorliegenden Erfindung betrifft die Verwendung von Naphthyl-azo-verbindungen mit der Formel I, 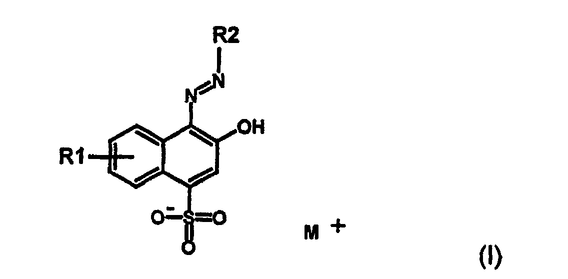
- in der R¹: für Wasserstoff, Halogen, Hydroxy, Nitro, eine C₁-C₄-Alkylgruppe, eine C₁-C₄-Alkoxygruppe, Sulfogruppe oder Carboxygruppe steht,
- R²: für eine 1- oder 2-Naphthylgruppe, die mindestens eine Hydroxygruppe trägt und als weitere Substituenten Hydroxy-, Sulfo-, Carboxy-, Nitrogruppen oder Halogenatome aufweisen kann, oder für einen 1-Aryl-5-hydroxy-4-pyrazolylrest, der in 3-Stellung durch eine C₁-C₄-Gruppe substituiert sein kann, steht,
M für Wasserstoff, ein Alkali- oder 1/2 Erdalkalimetall, 1/3 Al(III), 1/2 Zn(II), 1/4 Ti(IV), 1/4 Zr(IV), 1/3 Cr(III), 1/2 Co(II), 1/2 Fe(II), 1/3 Mo(III) bzw. 1/6 Mo(VI), 1/3 W(III) bzw. 1/6 W(VI) oder Ammonium steht,
als eine färbende Komponente in Haarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend Naphthyl-azo-Verbindungen mit der Formel I, 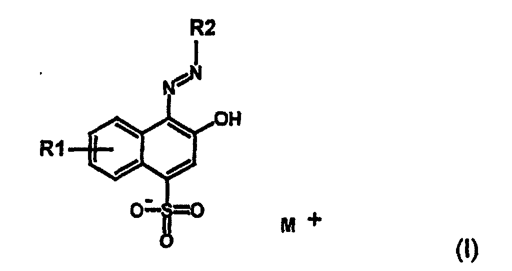
- in der R¹: für Wasserstoff, Halogen, Hydroxy, Nitro, eine C₁-C₄-Alkylgruppe, eine C₁-C₄-Alkoxygruppe, Sulfogruppe oder Carboxygruppe steht,
- R²: für eine 1- oder 2-Naphthylgruppe, die mindestens eine Hydroxygruppe trägt und als weitere Substituenten Hydroxy-, Sulfo-, Carboxy-, Nitrogruppen oder Halogenatome aufweisen kann, oder für einen 1-Aryl-5-hydroxy-4-pyrazolylrest, der in 3-Stellung durch eine C₁-C₄-Gruppe substituiert sein kann, steht,
- M: für Wasserstoff, ein Alkali- oder 1/2 Erdalkalimetall, 1/3 Al(III), 1/2 Zn(II), 1/4 Ti(IV), 1/4 Zr(IV), 1/3 Cr(III), 1/2 Co(II), 1/2 Fe(II), 1/3 Mo(III) bzw. 1/6 Mo(VI), 1/3 W(III) bzw. 1/6 W(VI) oder Ammonium steht,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Als weitere Komponenten können die oben genannten Metallsalze sowie die als Entwickler- und Kupplerkomponenten bekannten Verbindungen eingesetzt werden.

Die Naphthyl-azo-Verbindungen und die weiteren Komponenten können entweder gleichzeitig auf das Haar aufgebracht werden oder aber auch nacheinander, wobei es unerheblich ist, welche der Komponenten zuerst aufgetragen wird. Zwischen dem Auftragen der einzelnen Komponenten können bis zu 30 Minuten Zeitabstand liegen. Auch eine Vorbehandlung der Fasern mit einer Salzlösung ist möglich.

### Beispiele

### Herstellung einer Färbelösung

Es wurde 0,01 molare Lösungen der Verbindungen mit der Formel I hergestellt. Natriumacetat und ggf. ein weiteres Salz wurden equimolar zugegeben und mit einem Tropfen einer 20-%igen Fettalkylethersulfat-Lösung versetzt. Die Lösung wurde mit verdünnter Natronlauge oder Salzsäure auf einen pH-Wert von 6 eingestellt.

In diese Färbelösung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:

| | |
|---|---|
| - | keine oder eine sehr blasse Ausfärbung |
| (+) | schwache Intensität |
| + | mittlere Intensität |
| +(+) | mittlere bis starke Intensität |
| ++ | starke Intensität |
| ++(+) | starke his sehr starke Intensität |
| +++ | sehr starke Intensität |

**Tabelle 1**

| **Ausfärbungen mit Eriochrom®rot B** | | |
|---|---|---|
| **Metallsalz** | **Farbnuance** | **Intensität** |
| -- | Hellbraun | +(+) |
| Aluminiumlactat | Hellbraun | + |
| Aluminiumlactat* | Braunorange | ++ |
| Magnesiumchlorid | Hellbraun | + |
| Magnesiumchlorid* | Mittelbraun | ++ |
| Zinkchlorid | Hellbraun | +(+) |
| Zinkchlorid* | Orangebraun | ++ |

| | | |
|---|---|---|
| * In diesen Fällen wurden die Haarsträhnen zunächst 10 Minuten bei 30°C in die 1%ige Metallsalzlösung getaucht und mit saugfähigem Papier abgetupft. Anschließend wurden die Strähnen in eine Lösung, die die restlichen Bestandteile enthielt, getaucht. | | |

**Tabelle 2**

| Ausfärbungen mit Eriochrom®blauschwarz R | | |
|---|---|---|
| **Metallsalz** | **Farbnuance** | **Intensität** |
| -- | braunviolett | +(+) |
| Aluminiumlactat* | violett | ++ |
| Aluminiumlactat | violett | ++ |
| Magnesiumchlorid* | violett | ++ |
| Magnesiumchlorid | violettbraun | ++ |
| Zinkchlorid* | violett | ++ |
| Zinkchlorid | braunviolett | ++ |

### Anwendungsbeispiele

Alle Mengen-Angaben sind Gewichtsteile.

| 1. Färbecreme | |
|---|---|
| Hydrenol® D¹ | 1,0 |
| Lorol® techn.² | 1,0 |
| Akypo®Soft RLM 45N³ | 1,1 |
| p-Hydroxybenzoesäurepropylester | 0,05 |
| p-Hydroxybenzoesäuremethylester | 0,15 |
| Ammoniumsulfat | 1,0 |
| Eriochrom®blauschwarz R | 0,5 |
| Ammoniak (25 %-ig in Wasser) | ad pH 9 |
| Wasser | ad 100 |

| | |
|---|---|
| ¹ C16-18-Fettalkohol (INCI-Bezeichnung: Cetearyl alcohol) (COGNIS) | |
| ² C12-18-Fettalkohol (INCI-Bezeichnung: Coconut alcohol) (COGNIS) | |
| ³ Laurylalkohol+4,5 Ethylenoxid-Essigsäure-Natrium-Salz (ca. 82 % Aktivsubstanz in Wasser; INCI-Bezeichnung: Sodium Laureth-6 Carboxylate) (KAO) | |

Blonde Haare (Fa. Kerling) waren nach Behandlung mit dieser Färbecreme graumagenta.

| 2. Färbecreme | |
|---|---|
| Hydrenol® D | 1,0 |
| Lorol® techn. | 1,0 |
| Akypo®Soft RLM 45N | 1,1 |
| p-Hydroxybenzoesäurepropylester | 0,05 |
| p-Hydroxybenzoesäuremethylester | 0,15 |
| Ammoniumsulfat | 1,0 |
| Eriochrom®blauschwarz R | 0,2 |
| HC Blue No. 2 | 0,5 |
| Ammoniak (25 %-ig in Wasser) | ad pH 9 |
| Wasser | ad 100 |

Blonde Haare (Fa. Kerling) waren nach Behandlung mit dieser Färbecreme parmalila.

| 3. Färbecreme | |
|---|---|
| Hydrenol® D | 1,0 |
| Lorol® techn. | 1,0 |
| Akypo®Soft RLM 45N | 1,1 |
| p-Hydroxybenzoesäurepropylester | 0,05 |
| p-Hydroxybenzoesäuremethylester | 0,15 |
| Ammoniumsulfat | 1,0 |
| Eriochrom®blauschwarz R | 0,15 |
| HC Blue No. 2 | 0,15 |
| HC Red No.3 | 0,05 |
| N, N'-Bis(2-hydroxyethyl)-2-nitro-p-phenylendiamin | 0,45 |
| Ammoniak (25 %-ig in Wasser) | ad pH 7 |
| Wasser | ad 100 |

Blonde Haare (Fa. Kerling) waren nach Behandlung mit dieser Färbecreme dunkelpurpur.

| 4. Färbecreme | |
|---|---|
| Hydrenol® D | 6,0 |
| Lorol® techn. | 2,0 |
| Cremophor® A 25⁴ | 1,1 |
| Ammoniumsulfat | 1,0 |
| Eriochrom®blauschwarz R | 0,15 |
| HC Blue No. 2 | 0,15 |
| HC Red No.3 | 0,05 |
| N,N'-Bis(2-hydroxyethyl)-2-nitro-p-phenylendiamin | 0,45 |
| Ammoniak (25 %-ig in Wasser) | ad pH 7 |
| Wasser | ad 100 |

| | |
|---|---|
| ⁴ Talgalkohol + 25 Ethylenoxid (INCI-Bezeichnung: Ceteareth-25) (BASF) | |

Blonde Haare (Fa. Kerling) waren nach Behandlung mit dieser Färbecreme grauviolett.

| 5. Färbecreme | |
|---|---|
| Stenol® 1618⁵ | 8,1 |
| Paraffinöl perliqu. | 8,1 |
| Foryl®100⁶ | 0,6 |
| Dehyquart®A⁷ | 5,0 |
| Eriochrom®blauschwarz R | 0,5 |
| Ammoniak (25 %-ig in Wasser) | ad pH 9 |
| Wasser | ad 100 |

| | |
|---|---|
| ⁵ C16-18-Fettalkohol (INCI-Bezeichnung: Cetearyl alcohol) (COGNIS) | |
| ⁶ C12-18-Fettalkohol + 9 Ethylenoxid (INCI-Bezeichnung: Laureth-10) (COGNIS) | |
| ⁷ Trimethylhexadecylammoniumchlorid (ca. 25 % Aktivsubstanz in Wasser; INCI-Bezeichnung: Cetrimoniumchlorid) (COGNIS) | |

Blonde Haare (Fa. Kerling) waren nach Behandlung mit dieser Färbecreme graurosa.

## Patentansprüche

1. Mittel zum Färben von keratinhaltigen Fasern, insbesondere menschlichen Haaren, enthaltend als färbende Komponente mindestens eine Naphthylazo-verbindung mit der Formel I, 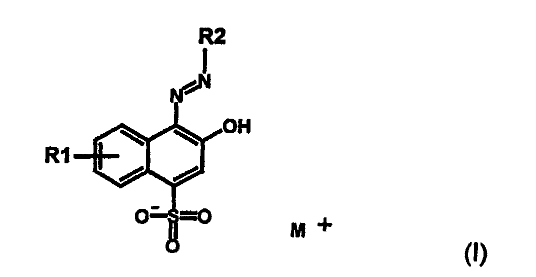
in der R¹ für Wasserstoff, Halogen, Hydroxy, eine C₁-C₄-Alkylgruppe, eine C₁-C₄-Alkoxygruppe, Sulfogruppe oder Carboxygruppe steht,
R² für eine 1- oder 2-Naphthylgruppe, die mindestens eine Hydroxygruppe trägt und als weitere Substituenten Hydroxy-, Sulfo-, Carboxy-, Nitrogruppen oder Halogenatome aufweisen kann, oder für einen 1-Aryl-5-hydroxy-4-pyrazolylrest, der in 3-Stellung durch eine C₁-C₄-Gruppe substituiert sein kann, steht,
M für Wasserstoff, ein Alkali- oder 1/2 Erdalkalimetall, 1/3 Al(III), 1/2 Zn(II), 1/4 Ti(IV), 1/4 Zr(IV), 1/3 Cr(III), 1/2 Co(II), 1/2 Fe(II), 1/3 Mo(III) bzw. 1/6 Mo(VI), 1/3 W(III) bzw. 1/6 W(VI) oder Ammonium steht.

2. Mittel nach Anspruch 1, **dadurch gekennzeichnet, daß** als Verbindungen mit der Formel I Eriochrom®blauschwarz R, Hydroxynaphtholblau, Acid Blue 158, Eriochrom®rot B, oder deren beliebigen Gemische eingesetzt werden.

3. Mittel nach einem der Ansprüche 1 bis 2, **dadurch gekennzeichnet, daß** die Naphthyl-azo-verbindungen mit der Formel I in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, daß** es zusätzlich ein oder mehrere Metallsalze der 2. oder 3. Hauptgruppe des Periodensystems und/oder ein oder mehrere Salze von Übergangsmetallen enthält.

5. Mittel nach Anspruch 4, **dadurch gekennzeichnet, daß** die Metallsalze ausgewählt sind aus der Gruppe der Chloride, Sulfate, Acetate, Lactate und Nitrate von Ca, Mg, Al(III), Zn(II), Ti(IV), Zr(IV), Cr(III), Co(II), Fe(II), Mo(III, VI), W(III, VI).

6. Mittel nach einem der Ansprüche 1 bis 5, **dadurch gekennzeichnet, daß** es zusätzlich Oxidationsfarbstoffvorprodukte und/oder direktziehende Farbstoffe aus der Gruppe der Nitrophenylendiamine, Nitroaminophenole, Anthrachinone oder Indophenole, vorzugsweise in einer Menge von 0,01 bis 20 Gew.-%, bezogen auf das gesamte Färbemittel, enthält.

7. Mittel nach einem der Ansprüche 1 bis 6, **dadurch gekennzeichnet, daß** es Oxidationsmittel, insbesondere H₂O₂, in einer Menge von 0,01 bis 6 Gew.-%, bezogen auf die Anwendungslösung, enthält.

8. Mittel nach einem der Ansprüche 1 bis 7, **dadurch gekennzeichnet, daß** es anionische, zwitterionische oder nichtionische Tenside enthält.

9. Verwendung von Naphthyl-azo-Verbindungen mit der Formel I, 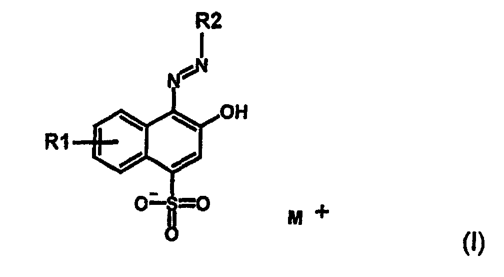
in der R¹ für Wasserstoff, Halogen, Hydroxy, Nitro, eine C₁-C₄-Alkylgruppe, eine C₁-C₄-Alkoxygruppe, Sulfogruppe oder Carboxygruppe steht,
R² für eine 1- oder 2-Naphthylgruppe, die mindestens eine Hydroxygruppe trägt und als weitere Substituenten Hydroxy-, Sulfo-, Carboxy-, Nitrogruppen oder Halogenatome aufweisen kann, oder für einen 1-Aryl-5-hydroxy-4-pyrazolylrest, der in 3-Stellung durch eine C₁-C₄-Gruppe substituiert sein kann, steht,
M für Wasserstoff, ein Alkali- oder 1/2 Erdalkalimetall, 1/3 Al(III), 1/2 Zn(II), 1/4
Ti(IV), 1/4 Zr(IV), 1/3 Cr(III), 1/2 Co(II), 1/2 Fe(II), 1/3 Mo(III) bzw. 1/6 Mo(VI), 1/3 W(III) bzw. 1/6 W(VI) oder Ammonium steht,
als eine färbende Komponente in Haarfärbemitteln.

10. Verfahren zum Färben von keratinhaltigen Fasern, insbesondere menschlichen Haaren, worin ein Färbemittel, enthaltend eine Naphthyl-azo-Verbindung mit der Formel I, 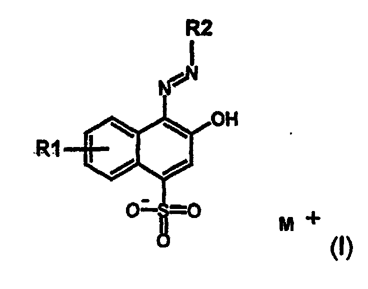
in der R¹ für Wasserstoff, Halogen, Hydroxy, Nitro, eine C₁-C₄-Alkylgruppe, eine C₁-C₄-Alkoxygruppe, Sulfogruppe oder Carboxygruppe steht,
R² für eine 1- oder 2-Naphthylgruppe, die mindestens eine Hydroxygruppe trägt und als weitere Substituenten Hydroxy-, Sulfo-, Carboxy-, Nitrogruppen oder Halogenatome aufweisen kann, oder für einen 1-Aryt-5-hydroxy-4-pyrazolylrest, der in 3-Stellung durch eine C₁-C₄-Gruppe substituiert sein kann, steht,
M für Wasserstoff, ein Alkali- oder 1/2 Erdalkalimetall, 1/3 Al(III), 1/2 Zn(II), 1/4 Ti(IV), 1/4 Zr(lV), 1/3 Cr(III), 1/2 Co(II), 1/2 Fe(II), 1/3 Mo(III) bzw. 1/6 Mo(VI), 1/3 W(III) bzw. 1/6 W(VI) oder Ammonium steht,
sowie übliche kosmetische Inhaltsstoffe auf die keratinhaltigen Fasern aufge bracht, einige Zeit, üblicherweise ca. 30 Minuten, auf der Faser belassen und anschließend wieder ausgespült oder mit einem Shampoo ausgewaschen wird.

## Claims

1. A composition for coloring keratin-containing fibers, more particularly human hair, **characterized in that** it contains as a coloring component at least one naphthylazo compound corresponding to formula (I): 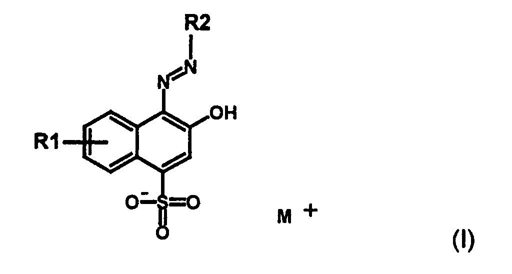 in which
R¹ is hydrogen, halogen, hydroxy, a C₁₋₄ alkyl group, a C₁₋₄ alkoxy group, sulfo group or carboxy group,
R² is a 1- or 2-naphthyl group which bears at least one hydroxy group and may carry hydroxy, sulfo, carboxy, nitro groups or halogen atoms as further substituents or a 1-aryl-5-hydroxy-4-pyrazolyl group which may be substituted in the 3-position by a C₁₋₄ group,
M is hydrogen, an alkali metal or 1/2 alkaline earth metal, 1/3 Al(III), 1/2 Zn(II), 1/4 Ti(IV), 1/4 Zr(IV), 1/3 Cr(III), 1/2 Co(II), 1/2 Fe(II), 1/3 Mo(III) or 1/6 Mo(VI), 1/3 W(III) or 1/6 W(VI) or ammonium.

2. A composition as claimed in claim 1, **characterized in that** Eriochrom®blauschwarz R, Hydroxynaphtholblau, Acid Blue 158, Eriochrom®rot B or mixtures thereof are used as the compounds of formula I.

3. A composition as claimed in claim 1 or 2, **characterized in that** the naphthylazo compounds corresponding to formula I are used in a quantity of 0.03 to 65 mmol and more particularly in a quantity of 1 to 40 mmol, based on 100 g of the colorant as a whole.

4. A composition as claimed in any of claims 1 to 3, **characterized in that** it additionally contains one or more metal salts of the 2nd or 3rd main group of the periodic system and/or one or more salts of transition metals.

5. A composition as claimed in claim 4, **characterized in that** the metal salts are selected from the group of chlorides, sulfates, acetates, lactates and nitrates of Ca, Mg, Al(III), Zn(II), Ti(IV), Zr(IV), Cr(III), Co(II), Fe(II), Mo(III,VI), W(III, VI).

6. A composition as claimed in any of claims 1 to 5, **characterized in that** it additionally contains oxidation dye precursors and/or substantive dyes from the group of nitrophenylenediamines, nitroaminophenols, anthraquinones or indophenols, preferably in a quantity of 0.01 to 20% by weight, based on the colorant as a whole.

7. A composition as claimed in any of claims 1 to 6, **characterized in that** it contains oxidizing agents, more particularly H₂O₂, in a quantity of 0.01 to 6% by weight, based on the solution applied.

8. A composition as claimed in any of claims 1 to 7, **characterized in that** it contains anionic, zwitterionic or nonionic surfactants.

9. The use of naphthylazo compounds corresponding to formula I: 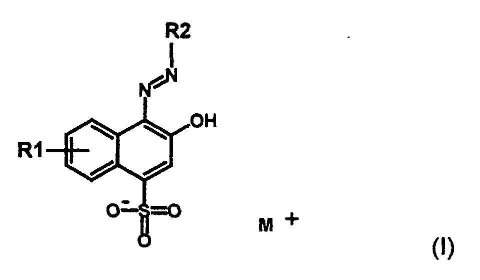 in which
R¹ is hydrogen, halogen, hydroxy, nitro, a C₁₋₄ alkyl group, a C₁₋₄ alkoxy group, sulfo group or carboxy group,
R² is a 1- or 2-naphthyl group which bears at least one hydroxy group and may carry hydroxy, sulfo, carboxy, nitro groups or halogen atoms as further substituents or a 1-aryl-5-hydroxy-4-pyrazolyl group which may be substituted in the 3-position by a C₁₋₄ group,
M is hydrogen, an alkali metal or 1/2 alkaline earth metal, 1/3 Al(III), 1/2 Zn(II), 1/4 Ti(IV), 1/4 Zr(IV), 1/3 Cr(III), 1/2 Co(II), 1/2 Fe(II), 1/3 Mo(III) or 1/6 Mo(VI), 1/3 W(III) or 1/6 W(VI) or ammonium. as a coloring component in oxidation hair colorants.

10. A process for coloring keratin-containing fibers, more particularly human hair, in which a colorant containing a naphthylazo compound corresponding to formula (I): 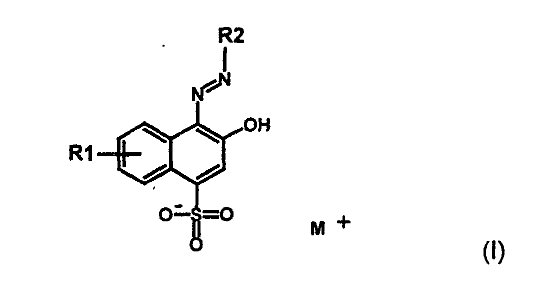 in which
R¹ is hydrogen, halogen, hydroxy, nitro, a C₁₋₄ alkyl group, a C₁₋₄ alkoxy group, sulfo group or carboxy group,
R² is a 1- or 2-naphthyl group which bears at least one hydroxy group and may carry hydroxy, sulfo, carboxy, nitro groups or halogen atoms as further substituents or a 1-aryl-5-hydroxy-4-pyrazolyl group which may be substituted in the 3-position by a C₁₋₄ group,
M is hydrogen, an alkali metal or 1/2 alkaline earth metal, 1/3 Al(III), 1/2 Zn(II), 1/4 Ti(IV), 1/4 Zr(IV), 1/3 Cr(III), 1/2 Co(II), 1/2 Fe(II), 1/3 Mo(III) or 1/6 Mo(VI), 1/3 W(III) or 1/6 W(VI) or ammonium,
and typical cosmetic ingredients is applied to the keratin-containing fibers, left thereon for a while, normally for about 30 minutes, and then rinsed out again or washed out with a shampoo.

## Revendications

1. Agent pour la coloration des fibres kératiniques, en particulier des cheveux humains, contenant comme composant colorant, au moins un composé naphtylazo de la formule I : 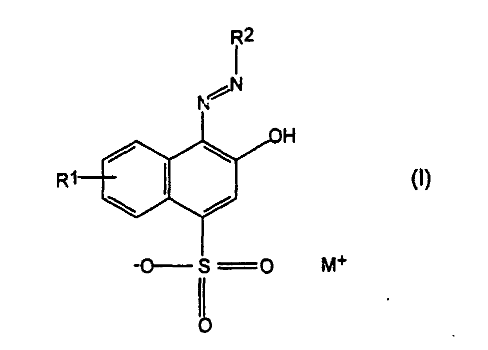 dans laquelle :
R¹ représente l'atome d'hydrogène, un atome d'halogène, le radical hydroxyle, un radical alcoyle en C₁-C₄, un radical alcoxyle en C₁-C₄, le radical sulfo ou le radical carboxyle ;
R² représente un radical 1- ou 2-naphtyle, qui porte au moins un radical hydroxyle et qui peut présenter comme autre substituant, un radical hydroxyle, sulfo, carboxyle, nitro ou un atome d'halogène, ou représente un reste 1-aryl-5-hydroxy-4-pyrazolyle, qui peut être substitué en position 3 par un radical en C₁-C₄ ;
M représente l'atome d'hydrogène, un métal alcalin ou 1/2 métal alcalino-terreux, 1/3 Al(III), 1/2 Zn(II), 1/4 Ti(IV), 1/4 Zr(IV), 1/3 Cr(III), 1/2 Co(II), 1/2 Fe(II), 1/3 Mo(III) ou 1/6 Mo(VI), 1/3 W(III) ou 1/6 W(VI) ou le radical ammonium.

2. Agent suivant la revendication 1, **caractérisé en ce que** l'on met en oeuvre comme composant ayant la formule I, l'Eriochrom® bleu-noir R, le bleu d'hydroxynaphtalène, le bleu acide 158, l'Eriochrom® rouge B ou leurs mélanges quelconques.

3. Agent suivant l'une quelconque des revendications 1 à 2, **caractérisé en ce que** les composés naphtylazo ayant la formule I sont présents en une quantité allant de 0,03 à 65 mmoles, en particulier de 1 à 40 mmoles, sur base de 100 g de l'agent colorant global.

4. Agent suivant l'une quelconque des revendications 1 à 3, **caractérisé en ce qu'**il contient en outre, un ou plusieurs sels métalliques des groupes principaux 2 ou 3 du système périodique et/ou un ou plusieurs sels des métaux de transition.

5. Agent suivant la revendication 4, **caractérisé en ce que** les sels métalliques sont choisis parmi le groupe des chlorure, sulfate, acétate, lactate et nitrate de Ca, Mg, Al(III), Zn(II), Ti(IV), Zr(IV), Cr(III), Co(II), Fe(II), Mo(III, VI), W(III, VI).

6. Agent suivant l'une quelconque des revendications 1 à 5, **caractérisé en ce qu'**il contient en outre, des précursseurs de coloration par oxydation et/ou des colorants se fixant directement parmi le groupe des nitrophénylènediamines, des nitroaminophénols, des anthraquinones ou des indophénols, de préférence en une quantité allant de 0,01 à 20% en poids, sur base de l'agent colorant global.

7. Agent suivant l'une quelconque des revendications 1 à 6, **caractérisé en ce qu'**il contient un agent d'oxydation, en particulier H₂O₂, en une quantité allant de 0,01 à 6% en poids, sur base de la solution d'application.

8. Agent suivant l'une quelconque des revendications 1 à 7, **caractérisé en ce qu'**il contient des tensioactifs anioniques, zwittérioniques ou non ioniques.

9. Utilisation de composé naphtylazo ayant la formule I : 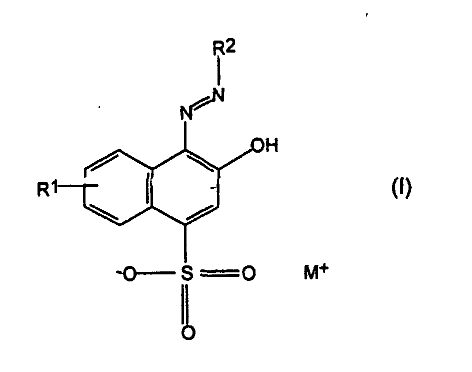 dans laquelle :
R¹ représente l'atome d'hydrogène, un atome d'halogène, le radical hydroxyle, le radical nitro, un radical alcoyle en C₁-C₄, un radical alcoxyle en C₁-C₄, le radical sulfo ou le radical carboxyle ;
R² représente un radical 1- ou 2-naphtyle, qui porte au moins un radical hydroxyle et qui peut présenter comme autre substituant, un radical hydroxyle, sulfo, carboxyle, nitro ou un atome d'halogène, ou représente un reste 1-aryl-5-hydroxy-4-pyrazolyle, qui peut être substitué en position 3 par un radical en C₁-C₄ ;
M représente l'atome d'hydrogène, un métal alcalin ou 1/2 métal alcalino-terreux, 1/3 Al(III), 1/2 Zn(II), 1/4 Ti(IV), 1/4 Zr(IV), 1/3 Cr(III), 1/2 Co(II), 1/2 Fe(II), 1/3 Mo(III) ou 1/6 Mo(VI), 1/3 W(III) ou 1/6 W(VI) ou le radical ammonium,
comme composant colorant dans des agents de coloration capillaire.

10. Procédé de coloration de fibres kératiniques, en particulier de cheveux humains, où l'on dispose sur les fibres kératiniques, un agent de coloration contenant un composé naphtylazo ayant la formule I : 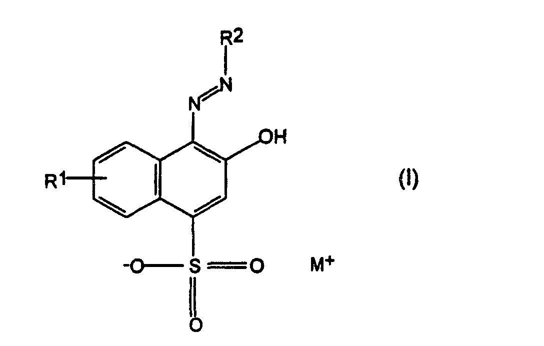 dans laquelle :
R¹ représente l'atome d'hydrogène, un atome d'halogène, le radical hydroxyle, le radical nitro, un radical alcoyle en C₁-C₄, un radical alcoxyle en C₁-C₄, le radical sulfo ou le radical carboxyle ;
R² représente un radical 1- ou 2-naphtyle, qui porte au moins un radical hydroxyle et qui peut présenter comme autre substituant, un radical hydroxyle, sulfo, carboxyle, nitro ou un atome d'halogène, ou représente un reste 1-aryl-5-hydroxy-4-pyrazolyle, qui peut être substitué en position 3 par un radical en C₁-C₄ ;
M représente l'atome d'hydrogène, un métal alcalin ou 1/2 métal alcalino-terreux, 1/3 Al(III), 1/2 Zn(II), 1/4 Ti(IV), 1/4 Zr(IV), 1/3 Cr(III), 1/2 Co(II), 1/2 Fe(II), 1/3 Mo(III) ou 1/6 Mo(VI), 1/3 W(III) ou 1/6 W(VI) ou le radical ammonium,
ainsi que les constituants cosmétiques usuels, on le laisse sur les fibres quelques temps, habituellement environ 30 minutes, et ensuite, on rince ou on lave avec un shampoing.
